(19) Europäisches Patentamt
European Patent Office
Office européen des brevets

(11) Publication number: 0 131 232 B1

(12) EUROPEAN PATENT SPECIFICATION

(45) Date of publication of patent specification: 23.12.87

(51) Int. Cl.$^4$: C 07 H 15/252

(21) Application number: 84107671.4

(22) Date of filing: 03.07.84

# (54) Stereoselective process for the preparation of anthracycline derivatives.

(30) Priority: 07.07.83 JP 123885/83

(43) Date of publication of application:
16.01.85 Bulletin 85/03

(45) Publication of the grant of the patent:
23.12.87 Bulletin 87/52

(84) Designated Contracting States:
CH DE FR GB LI

(56) References cited:
EP-A-0 014 853
EP-A-0 039 060
GB-A-2 002 754

JOURNAL OF ANTIBIOTICS, vol. 32, no. 10, October 1979, pages 1082-1084, Tokyo, JP; H. UMEZAWA et al.: "Tetrahydropyranyl derivatives of daunomycin and adriamycin"

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
4-23, Toyotama-kita
Nerima-ku Tokyo (JP)
Inventor: Takeuchi, Tomio
5-1-11, Higashi-gotanda
Shinagawa-ku Tokyo (JP)
Inventor: Tatsuta, Kuniaki
2-26-7, Matsunoki
Suginami-ku Tokyo (JP)
Inventor: Takahashi, Yoshikazu
3-2-3, Sakuragaoka
Tama-city Tokyo (JP)

(74) Representative: Meyer-Dulheuer, Karl-Hermann, Dr. et al
HOECHST Aktiengesellschaft Zentrale
Patentabteilung Postfach 80 03 20
D-6230 Frankfurt/Main 80 (DE)

EP 0 131 232 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a novel stereoselective process for preparing a 4'-O-tetrahydropyranyl derivative of an anthracycline compound having a daunosamine residue.

The anthracycline compounds of the formula I

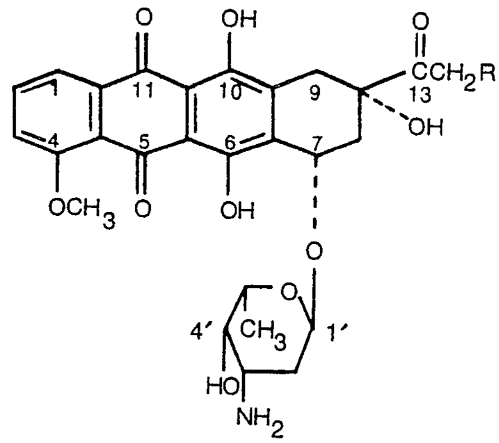

(I)

so far widely used in clinical practice as chemotherapeutic agents are daunomycin of said formula in which R represents a hydrogen atom, and adriamycin of said formula in which R represents a hydroxyl group. Although daunomycin and adriamycin show considerable potent anticancer activity, they are not entirely satisfactory. In an attempt to discover compounds having more potent anticancer activity, various analogues have been provided by various methods, such as fermentation, semi-synthesis, total synthesis and microbiological transformation. The present inventors have also provided useful derivatives of daunomycin and adriamycin which are compounds having a tetrahydropyranyl group at the 4'-position of the daunosamine residue, i.e.

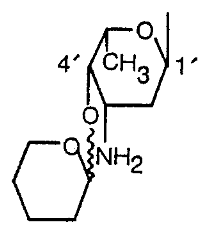

These 4'-O-tetrahydropyranyl compounds are, for example, described in JP—A1—56494/82 (Jap. Laid-Open Unexamined Patent Publication) and JP—A1—156 300/81 (Jap. Laid-Open Unexamined Patent Publication).

Such tetrahydropyranyl compounds include two isomers which are ascribed to the configuration at the C-2 position of the tetrahydropyran-2-yl group, as stated previously. One of the isomers shows a higher Rf value on silica gel thin-layer chromatography using chloroform-methanol (9:1) as a developer (hereinafter referred to as Compound (b)), and the other shows a lower Rf value on the same chromatography [hereinafter referred to as Compund (a)].

Compound (b), in particular, has been found to have much more potent anticancer activity against tumors in experimental animals than daunomycin and adriamycin. Its development as a drug for clinical practice has therefore been strongly desired.

According to the aforementioned known manufacturing methods, 4'-O-tetrahydropyranyl derivatives of anthracycline compounds are obtained as Compound (a) and Compound (b) each in an approximately equal amount. There has been a demand, therefore, for a process by which Compound (b), showing very potent anticancer activity, can be selectively obtained at a high yield from these isomers. It has now been found that tetrahydropyranyl derivatives can be obtained with an increased content of Compound (b) without any accompanying decrease in their total yields by using a metal-containing Lewis acid as the catalyst in place of an organic sulfonic acid.

This invention relates, therefore, to a process for preparing and selectively obtaining in a higher yield a stereoisomer showing a higher Rf value on silica gel thin-layer chromatography using chloroform-methanol (9:1) as a developer, among stereoisomers ascribed to the configuration at the C-2 position of the tetrahydropyran-2-yl group of a 4'-O-tetrahydropyranyl derivative of an anthracycline compound expressed by the formula (II)

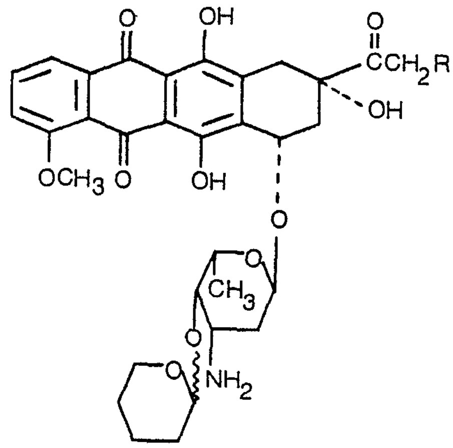

(II)

wherein R represents a hydrogen atom, a halogen atom or a protected hydroxyl group, which process comprises reacting an anthracycline compound of the formula (I)

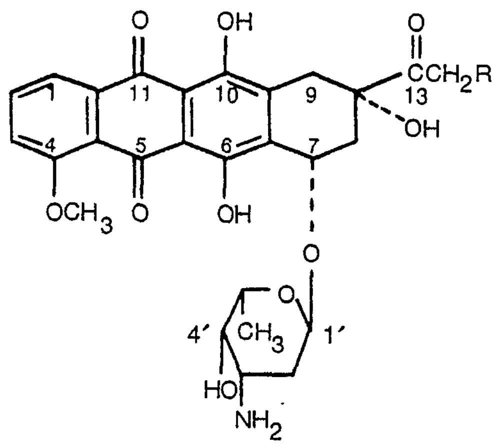

(I)

wherein R is as defined above, or an acid addition salt thereof, with 3,4-dihydro-2H pyran in an inert solvent in the presence of a metal-containing Lewis acid.

The process of the invention is applicable to anthracycline compounds having a daunosamine residue, such as daunomycins, adriamycins, carminomycins and feudomycins, and derivatives arising from their aglycones (see, for example, The Journal of Antibiotics, Vol. 14, 1596—1607, 1981).

Compounds preferred because of their anticancer activity as compounds of the formula (I) derived from daunomycin and adriamycin are, for example, those in which R represents a hydrogen atom, a halogen atom or a protected hydroxyl group. Specifically, there are cited compounds in which said halogen atom is a bromide atom (see, for instance, Japanese Laid-Open Unexamined Patent Publication No. 156300/81 quoted previously), and compounds in which said protected hydroxyl group is a group easily convertible into a hydroxyl group by mild hydrolysis, such as a lower alkanoyl group, an aralkylcarbonyl group, or an aromatic carbonyl group (see, for example, United States Patent No. 3,803,124). The latter compounds with the protected hydroxyl group as R should be those which can be converted into tetrahydropyranyl deriatives according to the process of this invention and then subjected to hydrolysis or hydrogenolysis to convert the R into a hydroxy group.

Examples of the metal-containing Lewis acids for use in the process of this invention are those halides of typical metals and transition metals (excluding alkali metals and alkaline earth metals) which can be used as Lewis acids. Their typical examples are $AlCl_3$, $AlBr_3$, $AlI_3$, $TiCl_4$, $FeCl_3$, $SnCl_4$ and $ZnCl_2$. Organometallic compounds which act as Lewis acids, such as $(C_2H_5)_2AlCl$ and $CH_3AlCl_2$, can also be used as the Lewis acids of this invention. Of these compounds, the use of $AlCl_3$, $TiCl_4$ and $AlBr_3$ are preferred to form Compound (b) with increased selectivity according to the present invention.

The inert solvents for use in the process of this invention may be any organic solvent which do not substantially affect the reaction for forming the tetrahydropyranyl derivative and which can form a uniform reaction mixture. Preferred solvents are, for example, aprotic polar solvents, such as N,N-dimethylformamide (hereinafter referred to as DMF), dimethyl sulfoxide, tetrahydrofuran, acetonitrile, and nitromethane. Suitable solvents can be selected by considering whether they can form a uniform reaction system, the properties of the starting compound of the formula (I) and the characters of Lewis acid used.

The reaction between the compound of formula (I) and 3,4-dihydro-2H-pyran of the formula

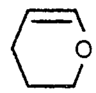

in the presence of the Lewis acid is performed in the above mentioned solvent advantageously at mild temperatures at which no substantial decomposition of the starting compounds takes place, preferably, at room temperature or below, specifically, temperatures ranging from −30 to 30°C. Under such conditions, the reaction is normally completed in a period of from 2 to 48 hours.

The amount of the metal-containing Lewis acid used in converting the compound of the formula (I) into its tetrahydropyranyl derivative can be varied widely depending on the type of the Lewis acid and the reaction conditions. However, it is used in 2 to 10 molar equivalents in general, preferably 4 to 6 molar equivalents, per mol of the compound of the formula (I).

The amount of 3,4-dihydro-2H-pyran for use in the formation of the tetrahydropyranyl derivative is not less than 10 molar equivalents, preferably 100 to 1000 molar equivalents, per mol of the compound of the formula (I).

After the reaction is completed, the compound of the formula (II), the tetrahydropyranyl derivative, can be subjected to isolation and purification steps [including resolution of isomers, i.e., Compound (a) and Compound (b)] by various methods that are known per se. For this purpose, methods that are frequently used in the isolation and purification of anthracycline antibiotics may be employed to isolate the intended compound from the reaction mixture. If the resulting compound has a halogen atom or a protected hydroxyl group as R, it may be hydrolyzed, if desired, without being isolated or purified, whereby a tetrahydropyranyl derivative of adriamycin can be obtained. (For details, see the aforecited patents.)

Example 1

Preparation of 4′-O-tetrahydropyran-2-yl-14-bromo-daunomycin

25 mg of 14-bromodaunomycin hydrobromide was dissolved in 2.5 ml of anhydrous DMF, and 25 mg of anhydrous aluminium chloride was added, followed by stirring the mixture for 1 hour at room temperature. During this period, the reaction mixture changed from red to purple.

Then, 1 ml of 3,4-dihydro-2H-pyran was added, and the resulting mixture was reacted for 18 hours with stirring at room temperature. Upon completion of the reaction, the reaction mixture was poured into 0.1M sodium bicarbonate containing disodium ethylenediaminetetraacetate [$Na_2$EDTA]. After stirring the mixture for several hours, it was extracted with ethyl acetate. The extract was dried over anhydrous sodium sulfate, and evaporated to dryness. The resulting crude product was separated chromatographically on a silica gel plate by using chloroform-methanol (9:1) as a developer. The separated spots of the desired compounds were scraped off the plate, and purified.

The yields of Compounds (a) and (b) were as follows:

| | |
|---|---|
| Compound (b) with an Rf value of 0.50 | 11 mg |
| Compound (a) with an Rf value of 0.33 | 4 mg |

Example 2

Preparation of 4′-O-tetrahydropyran-2-yl-daunomycin

The same procedure as in Example 1 was performed except that daunomycin was used as the starting material and aluminium chloride was used as the Lewis acid. 4′-O-tetrahydropyran-2-yl-daunomycin was obtained as Compounds (b) and (a).

Example 3

Preparation of 4′-O-tetrahydropyran-2-yl-daunomycin

The same procedure as in Example 1 was performed except that daunomycin was used as the starting material and ferric trichloride was used as the Lewis acid. 4′-O-tetrahydropyran-2-yl-daunomycin was obtained as Compounds (b) and (a).

Example 4

Preparation of 4′-O-tetrahydropyran-2-yl-14-bromodaunomycin

The same procedure as in Example 1 was performed using 14-bromodaunomycin hydrobromide as the starting material and aluminum tribromide as the Lewis acid.

4′-O-tetrahydropyran-2-yl-14-bromodaunomycin was obtained as Compounds (b) and (a).

Example 5

Preparation of 4′-O-tetrahydropyran-2-yl-14-bromodaunomycin

The same procedure as in Example 1 was performed using 14-bromodaunomycin as the starting material and titanium tetrachloride as the Lewis acid. 4′-O-tetrahydropyran-2-yl-14-bromodaunomycin was obtained as Compounds (b) and (a).

The following table shows the Lewis acid, solvent and reaction temperature used in Examples 2 to 5, as well as the proportion of Compound (b) to Compound (a) formed.

| Example No. | Lewis acid | Solvent | Temperature | (b)/(a) |
|---|---|---|---|---|
| 2 | $AlCl_3$ | DMF | Room temperature | 3/1 |
| 3 | $FeCl_3$ | DMF | Room temperature | 3/2 |
| 4 | $AlBr_3$ | DMF | Room temperature | 2/1 |
| 5 | $TiCl_4$ | DMF | Room temperature | 3/2 |

As described above, the 4'-O-tetrahydropyranyl derivatives of anthracycline compounds of the formula (II) prepared by the process of this invention consisted of Compounds (a) and (b), the ratio of Compound (a) to Compound (b) in terms of content being 1:1.5 to 1:3. Thus, this invention provides a process for preparing Compound (b), useful compound, selectively in a larger amount.

**Claims**

1. A process for preparing and selectively obtaining in a higher yield a stereoisomer showing a higher Rf value on silica gel thin-layer chromatography using chloroform/methanol (9:1) as developer among stereoisomers ascribed to the configuration on the C-2 position of the tetrahydropyran-2-yl group of a 4'-O-tetrahydropyranyl derivative of an anthracycline compound expressed by the formula

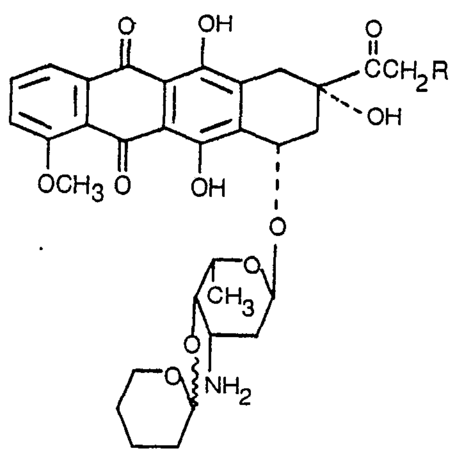

wherein R represents a hydrogen atom, a halogen atom or a protected hydroxyl group, which comprises reacting an anthracycline compound of the formula

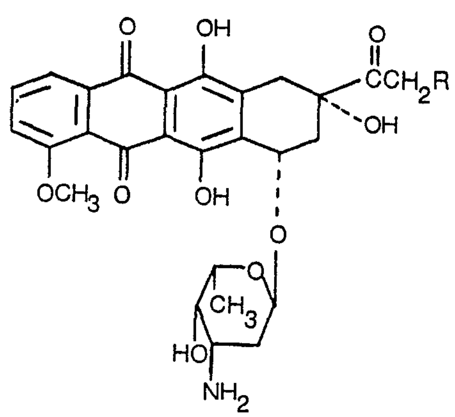

wherein R is as defined above, or an acid addition salt thereof, with 3,4-dihydro-2H-pyran in an inert solvent in the presence of a metal-containing Lewis acid, and isolating the desired stereoisomer by usual methods.

(2) The process of Claim (1), wherein the metal-containing Lewis acid is $AlCl_3$, $AlBr_3$, $TiCl_4$, or $FeCl_3$.

(3) The process of claims 1 and 2 wherein the desired isomer is isolated by chromatography.

**Patentansprüche**

1. Verfahren zur Herstellung und selektiven Gewinnung eines Stereoisomeren in höherer Ausbeute, das unter den Stereoisomeren bezüglich der Konfiguration an der C-2-Stellung der Tetrahydropyran-2-ylgruppe eines 4'-O-Tetrahydropyranylderivats einer Anthracyclinverbindun der Formel

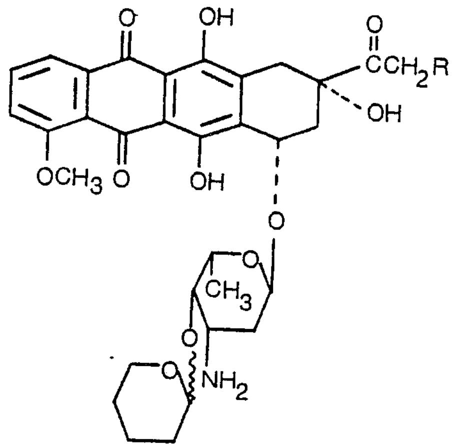

worin R für ein Wasserstoffatom, ein Halogenatom oder eine geschützte Hydroxylgruppe steht, bei Dünnschichtchromatographie an Kieselsäuregel mit Chloroform/Methanol (9:1) als Laufmittel einen höheren Rf-Wert zeigt, dadurch gekennzeichnet, dass man eine Anthracyclinverbindung der Formel

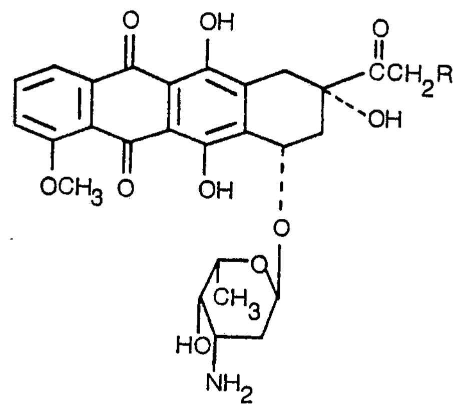

worin R die oben angegebene Bedeutung hat, oder ein Säureadditionssalz davon mit 3,4-Dihydro-2H-pyran in einem inerten Lösungsmittel in Gegenwart einer metallhaltigen Lewis-Säure umsetzt und das gewünschte Stereoisomer nach üblichen Methoden isoliert.

2. Verfahren nach Anspruch 1, worin die metallhaltige Lewis-Säure, $AlCl_3$, $AlBr_3$, $TiCl_4$ oder $FeCl_3$ ist.

3. Verfahren nach den Ansprüchen 1 und 2, worin das gewünschte Isomer durch Chromatographie isoliert wird.

**Revendications**

1. Procéde de préparation et d'obtention sélective, avec un rendement plus élevé, d'un stéréoisomère présentant une valeur de Rf plus élevée en chromatographie sur couche mince de gel de silica avec emploi d'un mélange chloroforme-méthanol (9:1) comme éluant parmi les stéréoisomères attribués à la configuration en position C-2 du groupe tétrahydropyranne-2-yle d'un dérivé 4'-O-tétrahydropyranylique d'un composé d'anthracycline représenté par la formule

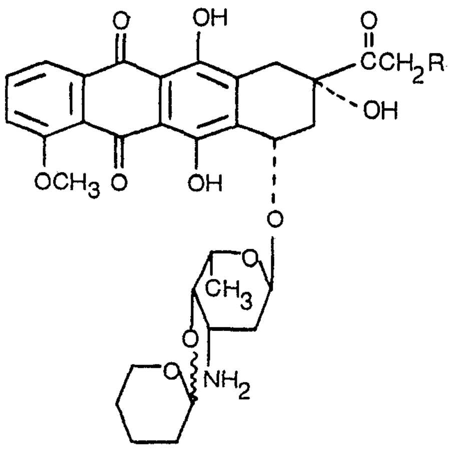

dans laquelle R représente un atome d'hydrogène, un atome d'halogène ou un groupe hydroxyle protégé, qui comprend la réaction d'un composé d'anthracycline de formule

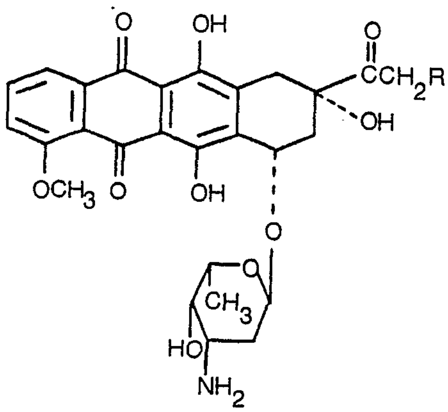

dans laquelle R est tel qu'il a été défini ci-dessus, ou d'un sel d'addition avec un acide de celui-ci, avec du 3,4-dihydro-2H-pyranne dans un solvant inerte en présence d'un acide de Lewis contenant un métal, et l'isolement du stéréoisomère désiré par des méthodes habituelles.

2. Procédé selon la revendication 1 dans lequel l'acide de Lewis contenant un métal est $AlCl_3$, $AlBr_3$, $TiCl_4$ ou $FeCl_3$.

3. Procédé selon les revendications 1 et 2 dans lequel l'isomère désiré est isolé par chromatographie.